Europäisches Patentamt

European Patent Office

Office européen des brevets

(11) Publication number: 0 251 745
A1

# EUROPEAN PATENT APPLICATION

(21) Application number: 87305733.5

(22) Date of filing: 26.06.87

(51) Int. Cl.⁴: A 61 N 1/06
A 61 N 1/40

(30) Priority: 27.06.86 JP 98428/86

(43) Date of publication of application:
07.01.88 Bulletin 88/01

(84) Designated Contracting States: DE FR GB IT

(71) Applicant: KUREHA KAGAKU KOGYO KABUSHIKI
KAISHA
9-11 Horidome-cho 1-chome Nihonbashi Chuo-ku
Tokyo (JP)

(72) Inventor: Kitagawa, Kiyoshi
2-503 Makuhari-Famiiru-Haitsu 5-417-16
Makuhari-cho Chiba-shi Chiba-ken (JP)

Onodera, Chikau
4-25-27 Kasuga-cho Nerima-ku
Tokyo (JP)

Onuma, Tadashi
2213-4 Takasaki Kukizaki-machi
Inashiki-gun Ibaraki-ken (JP)

Sogawa, Akira
105 Guroria-Haitsu 3-17-5 Toyotama-Minami
Nermia-ku Tokyo (JP)

(74) Representative: Senior, Alan Murray et al
J.A. KEMP & CO 14 South Square Gray's Inn
London WC1R 5EU (GB)

(54) A dielectric-heating electrode device for hyperthermia.

(57) An electrode device for heating endotract by an electric field with a high-frequency for use in the thermotherapy of cancers or the likes at the inside of a living body in which a high-frequency generating portion is surrounded with a flexible and gastight bag-like member and a coolant is filled to the inside of the bag-like member, wherein a gastight gas member having a function of supplying and discharging a gas into and out of the bag-like member is disposed to a portion on an outer circumference of the bag-like member.

Fig.3

**Description**

## A DIELECTRIC-HEATING ELECTRODE DEVICE FOR HYPERTHERMIA

This invention concerns a heating electrode device for medical use and, particularly, it relates to a high-fre quency heating electrode device for medical use that can be applied to hyperthermia such as for tumours.

High-frequency hyperthermia has been known in which therapy is carried out by applying heat to a lesional portion of a patient, utilizing the fact that cancer cells or the like are less resistant to heat than normal cells.

In the known high-frequency heating method, hyperthermia is carried out by putting a region including an aimed portion to be heated of a living body between two opposed plate-like electrodes and applying a high-frequency current between the electrodes from a high-frequency generator.

It has been difficult by this method to heat the aimed portion to be heated if it is situated deep inside, because portions other than the aimed portion to be heated may also possibly be heated since the high-frequency current flows substantially in parallel in the region between the opposed electrodes. Also a subcutaneous fat layer tends to be heated more intensely than endotract organ tissues due to the difference of electrical constants (electroconductivity, dielectric constant) between the subcutaneous fat layer and the endotract organ tissues, which may lead to patient's complaint of undesirable feeling of heat or risk of burn to epidermal tissues, etc.

As one of means for reducing the problems, a proposal has been made for the use of an endotract electrode of a structure in which one of the plate-like electrodes is replaced with a cylindrical or small electrode member, a flexible and gastight bag-like member closely in contact with a living body is attached to a portion of the electrode member that is in contact with or situated closer to a living body and a cooling medium (coolant) is filled in or circulated through the inside of the bag-like member (refer to Japanese Utility Model Application Laying-Open No. Sho 57-173712 and Japanese Patent Application Laying-Open No. Sho 60-119962). However, this lacks in general utilizability since it is necessary to design and use a bag-like member of a size corresponding to the inner diameter of the endotract organ for surely keeping the endotract electrode or the like to the aimed portion to be heated within a tubular endotract organ, for example, esophagus or stomach.

The present invention has been achieved in view of the foregoing and the object thereof is to provide a heating electrode device for medical use capable of surely and selectively heating a predetermined region situated deeply in a living body without giving much pain to a patient.

According to the present invention, the foregoing object can be attained in an endotract heating electrode device of applying high-frequency electromagnetic fields for the thermotherapy of cancers or the likes at the inside of a living body in which a high-frequency generating portion is surrounded with a flexible and gastight bag-like member and a coolant is filled to the inside of the bag-like member, wherein a gastight bag member having a function of supplying and discharging a gas is disposed to a portion at the outer circumference of the bag-like member.

The foregoing and other objects, as well as advantageous features of the present invention will be described more specifically while referring to the accompanying drawings, wherein

Figures 1 and 2 are general explanatory views of a high-frequency heating device for medical use;

Figure 3 is a detailed explanatory view for one embodiment of the electrode device according to the present invention;

Figure 4 is an explanatory cross sectional view taken along line I-I in Figure 3; and

Figure 5 is an explanatory view of a bag-like member shown in Figure 3 in an expanded state.

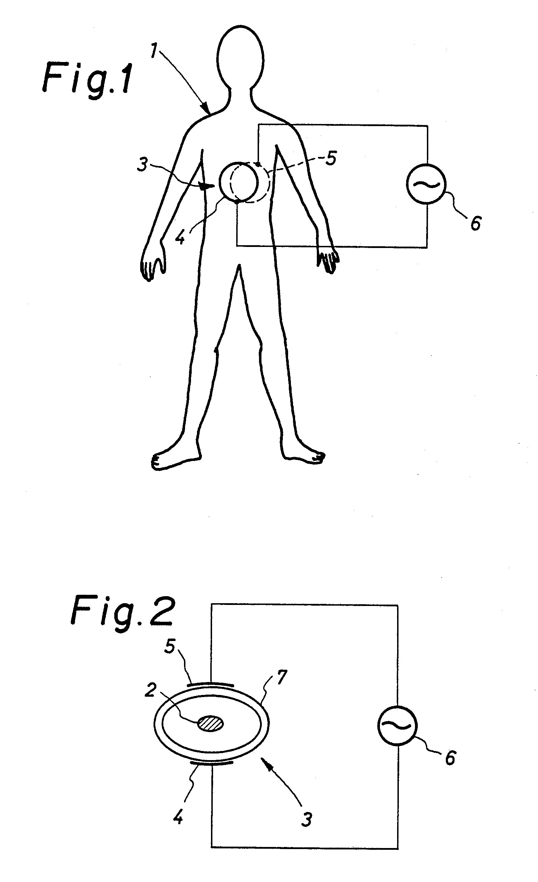

In the conventional high-frequency heating method, thermotherapy is carried out as shown in Figures 1 and 2, by putting a region 3 including an aimed portion 2 to be heated of a living body 1 between two opposed plate-like electrodes 4 and 5 and applying a high-frequency current between the electrodes 4 and 5 from a high-frequency generator 6.

It has been difficult by the method to heat the aimed portion 2 to be heated if it is situated at a deep inside, because those portions other than the aimed portion 2 to be heated may also possibly be heated since the high-frequency current flows substantially in parallel in the region between the opposed electrodes, as well as a subcutaneous fat layer 7 tends to be heated more intensely than endotract organ tissues due to the difference of electrical constants (electroconductivity, dielectric constant) between the subcutaneous fat layer and the endotract organ tissues, which may lead to patient's complaint of undesirable feeling of heat or risk of burn to epidermal tissues, etc.

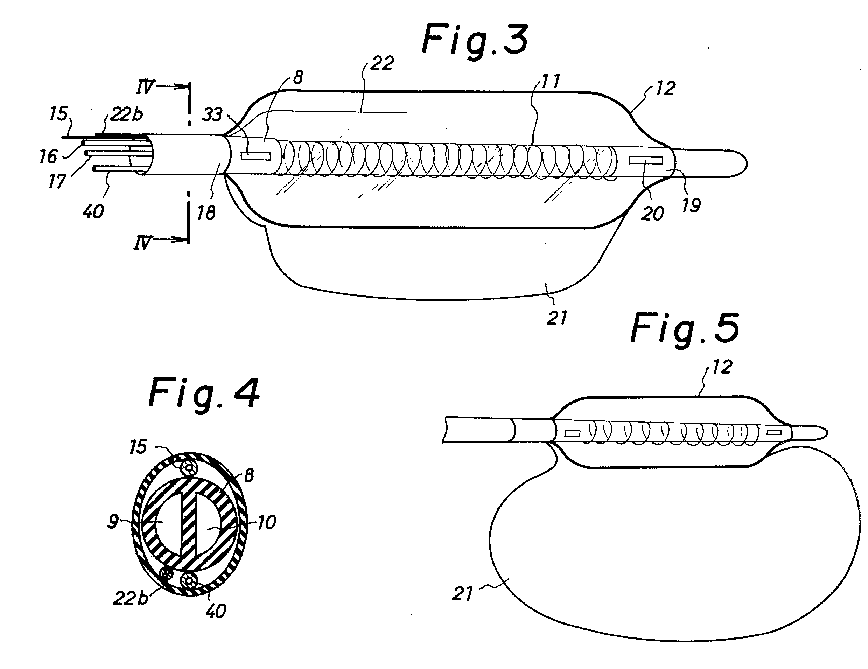

In Figures 3 through 5 showing an endotract electrode device as a preferred embodiment according to the present invention, reference numeral 8 represents a flexible two-channel tube made of silicone rubber in which a channel for supplying a coolant 9 and a channel for discharging the coolant 10 are integrally formed. The diameter of the channel tube 8 is desirably made smaller for easy access to a predetermined endotract organ. In a case where the electrode device is adapted to be applied to an esophagus, the tube 8 having, for example, about 3 - 8 mm of outer diameter is used. The tube 8 may have three or more coolant channels, and it may be made of other non-toxic and non-electroconductive flexible material than silicone rubber.

Reference numerals 16, 17 represent tubes for supplying and discharging the coolant to the supplying channel 9 and from the discharging channel 10, respectively.

At the top end side of the tube 8, there are attached a flexible high-frequency electrode 11 and a flexible bag-like member 12 of a size which approximates to the inner wall of the tubular endotract organ in the case where the bag-like member is expanded or not expanded. The tube 8 is provided with connectors (not illustrated) integrally connected to the coolant supplying tube 16 and the coolant discharging tube 17 respectively.

The high-frequency electrode 11 is secured to the outer circumference of the tube 8 and it may be composed, for example, of a helical metal wire turned back at its one end, with the turned back portion being in contact with a portion of the helical wire. It may be bellows, tubular braided member or the like so long as it has flexibility. The electrode 11 has any desired axial length and, for example, it is formed to such a length as equal to that of a tumour lesional portion.

A high-frequency lead wire 15 (for example, about 1 mm of outer diameter) is connected to an end on base side of the high-frequency electrode 11. The lead wire 15 is, for example, extended along the outer circumference of the two-channel tube 8 to a vicinity of the base of the tube 8, and a connector (not illustrated) for connection with a power source (not illustrated) is attached to the extended end of the wire.

The bag-like member 12 is formed into a cylindrical shape corresponding to the size and the shape of a tubular endotract near the lesional portion to which it is applied and, if desired, to the size and the shape of a portion narrowed by a tumour and the bag-like member 12 is secured to the outer circumference of the tube 8 at both of diametrically reduced ends 18 and 19 thereof so as to surround the electrode 11.

In a case where the electrode device is applied to an esophagus, a bag-like member 12, for example, having 5 - 30 mm of outer diameter and 30 - 140 mm of length is used. It is advantageous to attach a gastight bag member in the present invention as described later to a portion of the outer circumference of the bag-like member 12, since this can moderate the stringent requirement for accurately matching the outer diameter of the bag-like member 12 with the size of the portion to be heated.

The bag-like member 12 may be formed by shaping a plastic film or tube such as made of a flexible polyethylene or polypropylene membrane into a predetermined configuration. However, it is preferred to use a molded tube or balloon made of silicone rubber in view of the non-toxicity to a living body.

Reference numeral 22 represents a copper-constantan thermocouple as temperature detection means and the thermocouple 22 is secured by adhesives to the outer surface of the bag-like member 12 such that it can be in close contact with the endotract wall when the bag-like member 12 is expanded by the coolant. A plurality of thermocouples may be disposed circumferentially so that temperature is monitored at two or more positions.

Other thermocouples such as chromel-alumel may be used as the temperature detection means instead of copper-constantan or the thermo-couples may be replaced with thermisters, etc.

The lead wire 22b of the thermo-couple 22 is secured to the outer circumference of the tube 8 and connected with a connection connector (not illustrated). Reference 33 represents a coolant discharging port for communicating a space defined by the outer circumference of the tube 8 and the bag-like member 12 with the coolant discharging channel 10 of the tube 8, and reference numeral 20 represents a coolant supplying port for communicating the space as described above with the coolant supplying channel 9.

It may alternatively be adapted such that the coolant is supplied from the port 33 and discharged from the port 20. Further, the ports 20 and 33 may be disposed each by two or more to the tube 8.

"A coolant is filled to the inside of the bag-like member" in the present invention means a case of inserting the device of the present invention into an endotract with a coolant being previously filled to the inside of the bag-like member thereof, a case of inserting the device according to the present invention to the inside of the endotract and then supplying the coolant by a predetermined amount to the inside of the bag-like member thereby filling the inside with the coolant, as well as a case of supplying the coolant through the coolant supplying channel to the inside of the bag-like member and then discharging the coolant through the coolant discharging channel thereby circulating the coolant through the inside of the bag-like member.

Reference numeral 21 represents a gastight bag member disposed to a portion on the outer circumference of the bag-like member 12, so that air or like other gas is introduced therein by way of a introducing tube 40. The gastight bag member 21 is bonded to the bag-like member 12, for example, by means of adhesives. The use of a gas is advantageous as compared with the use of water or like other liquid in that intrusion of high-frequency electric current to the side of the gastight bag member is decreased as less as possible. The gastight bag member 21 may be formed by shaping a film or tube made of flexible polyethylene or polypropylene into a predetermined configuration. However, the use of a molding body or balloon made of silicone rubber or natural rubber which can be extended or expanded readily by the introduction of a gas is desirable in view of the general utilizability.

Although the channel for flowing gas to the inside of the gastight bag member is illustrated in Figure 3 as of a one path structure, a plurality of gas flowing channels may be formed. A portion on the circumference of the bag-like member 12 means herein a portion or the entire part in the circumferential or the axial direction of the bag-like member 12 excepting for the aimed portion to be heated.

The electrode device according to the present invention is inserted to the inside of the tract such as an esophagus or stomach in a state where the bag-like member 12 and the gastight bag member 21 is deflated. After the insertion, a coolant is charged and circulated to and through the inside of the bag-like member 12 through the coolant supplying and discharging tubes 16 and 17 to expand the bag-like member 12. Then, when air is injected through the introducing tube 40 into the inside of the gastight bag member 21, the bag member 21 is expanded, by which the electrode can surely be disposed within the tract under an appropriate pressure. The electrode device according to the present invention can attain the purpose of the thermotherapy in co-operation with an external electrode device. When the electrode device according to present invention is combined with the external electrode device which comprises an external electrode (non-sensitive electrode) having an electrode area greater than ten times as compared with that of the electrode device according to present invention, the aimed portion to be heated within the tract can effectively be healed.

## Claims

1. An electrode device for endotract heating by an electric field with a high frequency for use in the thermotherapy of cancers or the like at the inside of a living body, the device having a high-frequency generating portion (11) surrounded with a flexible and gastight bag-like member (12) means (16, 17) to supply coolant to, and withdraw it from, the inside said bag-like member, and including a gastight bag member (21) having means (40) for supplying and discharging a gas thereto disposed on a portion of the outside of said bag-like member (12).

2. An electrode device as defined in claim 1, wherein the high-frequency generating portion comprises a flexible support member (8) and a flexible electrode (11) supported with said support member, one end of said flexible electrode being situated on a side of one end of said support member.

3. An electrode device as defined in claim 2, wherein the flexible electrode comprises one of a helical metal wire, a bellow and a tubular braided member.

4. An electrode device as defined in claim 2 or 3 wherein the support member (8) comprises a two-channel tube in which a channel for supplying a coolant and a channel for discharging a coolant are formed integrally.

5. An electrode device as defined in claim 4, wherein said two-channel tube has coolant supply and discharge ports communicating the space between the outside of the support member and inside of the bag-like member with the coolant supply and discharging channels respectively.

6. An electrode device as defined in any preceding claim, wherein the flexible bag-like member is made of a high molecular polymer and has a size approximate to that of an inner wall of a tubular endotract organ.

7. An electrode device as defined in any preceding claim including a temperature detection means secured on an outer surface of the flexible bag-like member, to be brought into close contact with the tract wall when the flexible bag-like member is expanded by the coolant.

8. An electrode device as defined in any preceding claim wherein the gastight bag member comprises a molding body or balloon made of synthetic or natural rubber, which is readily extended or expanded by the introduction of the gas.

9. An electrode device as defined in any preceding claim wherein the gastight bag member is disposed on a portion of or on an entire circumferential or axial part of the bag-like member except for a portion corresponding to the aimed portion to be heated of the living body.

10. An electrode device as defined in any preceding claim wherein a tube for introducing and discharging the gas leads to the gastight bag member.

11. An electrode device as defined in any preceding claim, wherein the gastight bag member is bonded to the bag-like member by means of an adhesive.

0251745

Fig.1

Fig.2

# Fig.3

# Fig.4

# Fig.5

0251745

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 115 420  (KUREHA KAGAKU KOGYO) <br> * Figures 11,12; page 11, line 4 - page 18, line 26 * | 1-6,8-11 | A 61 N    1/06 <br> A 61 N    1/40 |
| Y | SOVIET INVENTIONS ILLUSTRATED, section P/Q, week 8502, P34, abstract no. 85-010720/02, 20th February 1985, Derwent Publications Ltd, London, GB; & SU-A-1 093 347 (TSELINOGRAD MED. INS.) 23-05-1984 <br> *   Class   P34,   page   6,   no. 85-010720/02 * | 1-6,8-11 | |
| A | EP-A-0 034 516  (SOCIETE ANONYME DE TELECOMMUNICATIONS) <br> * Figure 2; page 8, line 15 - page 9, line 14 * | 1-3 | |
| A | DE-A-2 407 559  (DORNIER) <br> * Figure; claims 1-4 * | 4-7 | TECHNICAL FIELDS SEARCHED (Int. Cl.4) <br><br> A 61 N |
| A | EP-A-0 139 433  (KUREHA KAGAKU KOGYO) <br> * Figure 15; claims 1-10 * | | |

The present search report has been drawn up for all claims

| Place of search | Date of completion of the search | Examiner |
|---|---|---|
| THE HAGUE | 30-09-1987 | DELEU A.J.H. |